(19) Europäisches Patentamt
European Patent Office
Office européen des brevets

(11) **EP 4 512 797 A1**

(12) **EUROPEAN PATENT APPLICATION**
published in accordance with Art. 153(4) EPC

(43) Date of publication:
**26.02.2025 Bulletin 2025/09**

(21) Application number: **23791209.2**

(22) Date of filing: **18.04.2023**

(51) International Patent Classification (IPC):
***C07C 219/10*** (2006.01) ***C07C 217/08*** (2006.01)
***C07C 323/58*** (2006.01) ***C07C 219/16*** (2006.01)
***C07C 229/12*** (2006.01) ***C07D 209/48*** (2006.01)
***A61K 31/216*** (2006.01) ***A61K 31/4035*** (2006.01)
***A61K 31/223*** (2006.01) ***A61K 31/265*** (2006.01)
***A61K 31/22*** (2006.01) ***A61P 29/00*** (2006.01)

(52) Cooperative Patent Classification (CPC):
**C07C 219/10; A61K 31/216; A61K 31/22; A61K 31/223; A61K 31/265; A61K 31/4035; A61P 29/00; C07C 217/08; C07C 229/12; C07C 323/58; C07D 207/404;** C07B 2200/07; C07C 2601/08

(86) International application number:
**PCT/CN2023/088864**

(87) International publication number:
**WO 2023/202554 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.04.2022 CN 202210408886**

(71) Applicant: **SHIJIAZHUANG DISCOVERY MEDICINE TECHNOLOGY CO., LTD**
**Shijiazhuang, Hebei 050035 (CN)**

(72) Inventors:
- **MENG, Yuelei**
  **Shijiazhuang, Hebei 050035 (CN)**
- **WANG, Lu**
  **Shijiazhuang, Hebei 050035 (CN)**
- **GAO, Liang**
  **Shijiazhuang, Hebei 050035 (CN)**

(74) Representative: **Engelhard, Markus**
**Boehmert & Boehmert Anwaltspartnerschaft mbB**
**Pettenkoferstrasse 22**
**80336 München (DE)**

# (54) CHIRAL ARYL PROPIONIC ACID DERIVATIVE AND PHARMACEUTICAL COMPOSITION THEREOF, AND USE

(57) Disclosed are a chiral aryl propionic acid derivative and a pharmaceutical composition thereof, and a use. The chiral aryl propionic acid derivative is as shown in formula (I), and the definition of each group is given in the description. The chiral aryl propionic acid derivative has antipyretic, analgesic and anti-inflammatory effects. The compound of the present invention has high activity and can be locally administrated, a reduced dosage is expected, and adverse reactions are reduced.

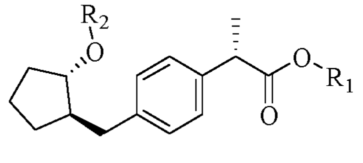

(I)

EP 4 512 797 A1

## Description

### TECHNICAL FIELD

The present invention relates to but is not limited to the technical field of pharmaceutical chemistry, in particular to a chiral aryl propionic acid derivative and a pharmaceutical composition thereof, and a use.

### BACKGROUND

Loxoprofen sodium with a chemical name of sodium 2-[4-(2-oxocyclopentyl-1-ylmethyl)phenyl] propionate is the first propionic acid precursor non-steroidal anti-inflammatory drugs (NSAIDs), which is developed by Japan Sankyo Corporation. Loxoprofen Sodium Tablets were launched in Japan in July 1986 under the trade name Loxonin, and marketed in China in 1999 under the original Chinese trademark name "Le Song". The dosage forms of the marketed drugs are tablets, capsules, fine particles, patches and gels. Among various dosage forms of marketed drugs, the API is present in a form of loxoprofen sodium dihydrate.

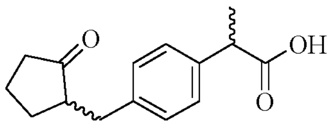

Loxoprofen

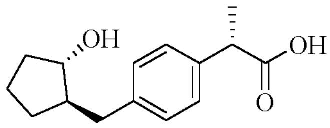

trans-OH metabolite

Loxoprofen is a prodrug, which has weak activity and is metabolized by liver after oral administration to transform loxoprofen into an active ingredient so as to exert its therapeutic effect. Loxoprofen is considered as being metabolized by a carbonyl reductase in skins and subcutaneous muscle tissues after local administration. Loxoprofen has 2 chiral centers and 4 chiral isomers. At present, loxoprofen sodium is marketed in a racemic form. There are no literatures reporting differences in pharmacological effects among four isomers of loxoprofen. After being orally administrated, loxoprofen is rapidly absorbed. After 30-50 minutes, the plasma concentrations of loxoprofen and its metabolic products reach peaks, and plasma protein binding rates are 97% and 93%, respectively; after single local administration of 1% loxoprofen (100 mg), 10% of dose is transferred to a body in 12 hours.

Carbonyl is reduced to hydroxyl upon loxoprofen is metabolized, and 3 chiral centers and 8 isomers will be comprised in the structure at this point, among them a trans-OH metabolite is one of the metabolites. For in-vivo metabolites containing 3 chiral centers, there are no literatures reporting the differences in specific pharmacological effects of the corresponding diastereoisomers.

Detailed researches have been conducted on several isomers, and an efficient fast-acting non-steroidal anti-inflammatory drug derivative has been developed through structural modifications on this basis.

### SUMMARY

The inventors develop a chiral aryl propionic acid derivative. The compound has antipyretic, analgesic and anti-inflammatory effects. The compound of the present invention has a better anti-rheumatoid arthritis effect, and it is expected to reduce the administration dose and lower adverse reactions. In addition, it is unexpectedly found that the chiral aryl propionic acid derivative is highly distributed in joints. Further studies have shown that when being externally applied to skins, the chiral aryl propionic acid derivative has better joint fluid distribution and better therapeutic effects, and can be used as a locally administrated non-steroidal anti-inflammatory drug.

One aspect of the present invention provides a chiral aryl propionic acid derivative as shown in formula (I), and a tautomer, a solvate or a pharmaceutically acceptable salt thereof:

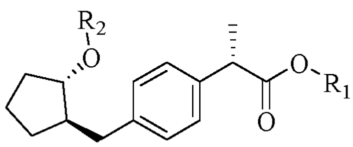

(I)

in the formula (I), $R_1$ is H, or is selected from

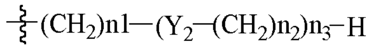

substituted by one or more

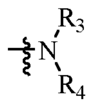
or
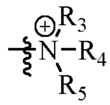

or unsubstituted

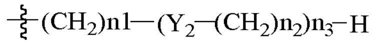
;

$R_2$ is H, or is selected from

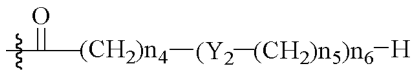

substituted by one or more

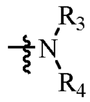
or
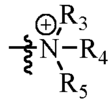

or unsubstituted

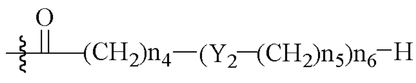
;

and when $R_1$ is H, $R_2$ is not H;
$Y_2$ is O, or $N(R_6)$, or S;
$n_1$, $n_2$, $n_3$, $n_4$, $n_5$ and $n_6$ are each independently 0, 1, 2, 3, 4, 5, 6, 7, 8, 9, and 10;
$R_3$, $R_4$ and $R_5$ are each independently H, C1-C20 hydrocarbyl, or C1-C20 alkyl carbonyl, or $R_3$ and $R_4$ form a ring together with a nitrogen atom to which they are attached;
$R_6$ is H, or C1-C20 hydrocarbyl;
$A^-$ represents an acceptable inorganic or organic anion.

In the embodiments of the present application, the hydrocarbyl comprises an alkly hydrocarbyl, an alkenyl hydrocarbyl, or an alkynyl hydrocarbyl, and also comprises a heterocyclyl, an aryl, or a heteroaryl.

In the embodiments of the present application, the hydrocarbyl comprises a straight, branched or cyclic hydrocarbyl.

In the embodiments of the present invention, the C1-C20 hydrocarbyl refers to a saturated or unsaturated aliphatic hydrocarbyl containing 1 to 20 carbon atoms, including C1-C20 alkyl hydrocarbyl, C2-C20 alkenyl hydrocarbyl, and C2-C20 alkynyl hydrocarbyl. For example, the C1-C20 hydrocarbyl includes but is not limited to methyl, ethyl, propyl, isopropyl, butyl, sec-butyl, tert-butyl, pentyl, hexyl, cyclopropyl, cyclobutyl, cyclopentyl, cyclohexyl, ethenyl, ethynyl, propenyl, propynyl, allyl, 2-methyl-2-butenyl, 2-butenyl ($-CH_2-CH=CH-CH_3$), 3-butenyl ($-CH_2-CH_2-CH=CH_2$), 4-pentenyl ($-CH_2-CH_2-CH_2-CH=CH_2$), 2-methyl-2-pentenyl ($-CH_2-C(CH_3)=CH-CH_2-CH_3$) and 5-hexenyl ($-CH_2CH_2CH_2CH=CH_2$).

In the embodiments of the present application, the hydrocarbyl comprises a C2-C20 heterocyclyl. The heterocyclyl means that this heterocycle at least comprises one or more atoms selected from oxygen, nitrogen and sulfur; and includes, but is not limited to: oxiranyl, aziridinyl, thiiranyl, azetidinyl, oxetanyl, thietanyl, 1,2-dithietanyl, 1,3-dithietanyl, pyrrolidinyl, dihydro-1H-pyrrolyl, dihydrofuranyl, tetrahydrofuranyl, dihydrothienyl, tetrahydrothienyl, imidazolidinyl, piperidyl, piperazinyl, isoquinolyl, tetrahydroisoquinolyl, morpholinyl, thiomorpholinyl, 1,1-dioxo-thiomorpholinyl, dihydropyranyl, tetrahydropyranyl, hexahydrothiapyranyl, hexahydropyrimidinyl, oxazacyclohexyl, thiazanyl, thioxanyl, homopiperazinyl, homopiperidyl, azepanyl, oxepanyl, thiepanyl, oxazepanyl, diazepanyl, 1,4-diazepanyl, thiazetanyl, tetrahydro thiapyranyl, oxazolidinyl, thiazolidinyl, isothiazolidinyl, 1,1-dioxoisothiazolidinonyl and oxazolidinonyl.

**In** the embodiments of the present application, the hydrocarbyl comprises aryl including but not limited to: benzene, naphthalene, anthracene, or biphenyl, etc.

**In** the embodiments of the present application, the hydrocarbyl comprises heteroaryl including but not limited to: pyrimidine, furan, thiazole, thiophene, pyridine, pyrrole and imidazole.

**In** the embodiments of the present application, the pharmaceutically acceptable salt refers to any form of chiral aryl propionic acid derivative used according to the present invention, wherein the chiral aryl propionic acid derivative is in an ion form or charged, and is coupled with counter ions (negative ions or positive ions) or is in a solution;

In the embodiments of the present application, the pharmaceutically acceptable salt comprises salts formed by the chiral aryl propionic acid derivative of the present invention and negative ions, the negative ions include but are not limited to: fluoride ions, chloride ions, bromide ions, iodide ions, acetate ions, benzoate ions, citrate ions, tartrate ions, oxalate ions, malate ions, ascorbate ions, and fumarate ions, etc.;

In the embodiments of the present application, the pharmaceutically acceptable salt includes an inner salt formed by the chiral aryl propionic acid derivative in the present invention, and the inner salt refers to a salt formed within a molecule when the molecule contains both carboxyl and amino, indicating that the same molecule carries both positive and negative charges;

In the embodiments of the present application, the pharmaceutically acceptable salt comprises a salt formed between the molecules of the chiral aryl propionic acid derivative of the present invention. The salt formed between the molecules refers to a salt formed between different molecules when the molecule of the chiral aryl propionic acid derivative contains both carboxyl and amino.

In some embodiments, $R_1$ is hydrogen, and $R_2$ is

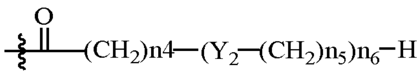

substituted by one or more

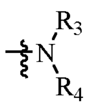 or 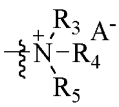

or unsubstituted

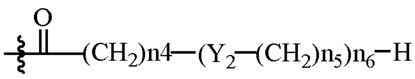;

In some embodiments, $R_2$ is hydrogen, and $R_1$ is $(CH_2)n1\text{-}(Y_2\text{-}(CH_2)n_2)n_3\text{-}H$ substituted by one or more

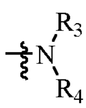 or 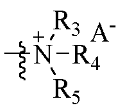

or unsubstituted

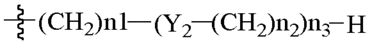;

In some embodiments, $R_1$ is $(CH_2)n1\text{-}(Y_2\text{-}(CH_2)n_2)n_3\text{-}H$ substituted by one or more

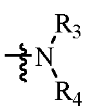 or 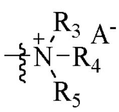,

and $R_2$ is

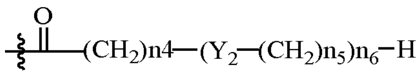

substituted by one or more

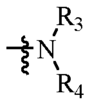 or 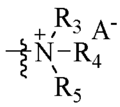 ;

In some embodiments, $R_1$ is

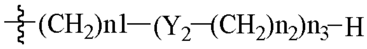

substituted or unsubstituted by one or more

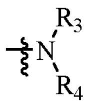 or 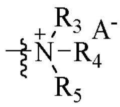 ,

and $R_2$ is

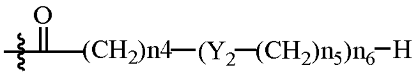 ;

In some embodiments, $R_1$ is

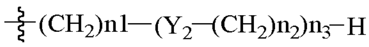 ,

and $R_2$ is

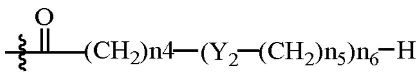

substituted by one or more

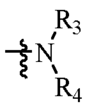 or 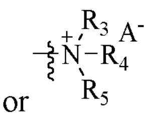 ;

In some embodiments, when $R_1$ or $R_2$ is not hydrogen, its substituent

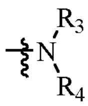

or

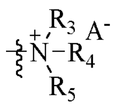

may replace the hydrogen on the terminal carbon, or the hydrogen on the non-terminal carbon.

In some embodiments, $R_3$ and $R_4$ are both H;

In some embodiments, $R_3$ and $R_4$ are both C1-C20 hydrocarbyl, or C1-C20 alkyl carbonyl; preferably, $R_3$ and $R_4$ are both C1-C6 hydrocarbyl, or C1-C6 alkyl carbonyl; more preferably, $R_3$ and $R_4$ are both C1-C6 alkyl;

In some embodiments, $R_3$ is H, and $R_4$ is C1-C20 hydrocarbyl, or C1-C20 alkyl carbonyl; preferably, $R_4$ is both C1-C6 hydrocarbyl; more preferably, $R_4$ is C1-C6 alkyl;

In some embodiments, $R_4$ is H, and $R_3$ is C1-C20 hydrocarbyl, or C1-C20 alkyl carbonyl; preferably, $R_3$ is both C1-C6 hydrocarbyl; more preferably, $R_3$ is C1-C6 alkyl;

In some embodiments, $R_3$ and $R_4$ form a ring together with a nitrogen atom to which they are attached, wherein the nitrogen atom can be attached to $R_3$ and/or $R_4$ via a common carbon-nitrogen bond, or via an amide bond; the ring formed by $R_3$ and $R_4$ together with the nitrogen atom to which they are attached can be a 4-membered ring, a 5-membered ring, a 6-membered ring, or a 7-membered ring.

In some embodiments, $R_3$, $R_4$ and $R_5$ are all H;

In some embodiments, $R_3$ and $R_4$ are both C1-C20 hydrocarbyl or C1-C20 alkyl carbonyl, and $R_5$ is H; preferably, $R_3$ and $R_4$ are both C1-C6 hydrocarbyl or C1-C6 alkyl carbonyl, and $R_5$ is H; more preferably, $R_3$ and $R_4$ are both C1-C6 alkyl, and $R_5$ is H;

In some embodiments, $R_3$, $R_4$ and $R_5$ are all C1-C20 hydrocarbyl or C1-C20 alkyl carbonyl; preferably, $R_3$, $R_4$ and $R_5$ are all C1-C6 hydrocarbyl; more preferably, $R_3$, $R_4$ and $R_5$ are all C1-C6 alkyl;

In some embodiments, $R_3$ and $R_4$ form a ring together with a nitrogen atom to which they are attached, and $R_5$ is both H or C1-C20 hydrocarbyl, wherein, the nitrogen atom and $R_3$ and/or $R_4$ can be attached via a common carbon-nitrogen bond, or via an amide bond; the ring formed by $R_3$ and $R_4$ together with the nitrogen atom to which they are attached can be a 4-membered ring, a 5-membered ring, a 6-membered ring, or a 7-membered ring.

In some embodiments, $Y_2$ is O, or $N(R_6)$, or S;

In some embodiments, $Y_2$ is $N(R_6)$, or S.

In some embodiments, $Y_2$ is $N(R_6)$, or O.

In some embodiments, $Y_2$ is O, or $N(R_6)$, or S.

In some embodiments, $R_6$ is H.

In some embodiments, $R_6$ is C1-C20 hydrocarbyl; preferably, $R_6$ is C1-C6 hydrocarbyl; more preferably, $R_6$ is C1-C6 alkyl.

In some embodiments, ni, $n_2$ and $n_3$ are each independently 0, or 1, or 2, or 3, or 4, or 5, or 6, or 7, or 8, or 9, or 10, preferably, $n_1$, $n_2$ and $n_3$ are each independently 0, or 1, or 2, or 3, or 4;

In some embodiments, $n_1$, $n_2$ and $n_3$ are all 0.

In some embodiments, $n_4$, $n_5$ and $n_6$ are each independently 0, or 1, or 2, or 3, or 4, or 5, or 6, or 7, or 8, or 9, or 10, preferably, $n_1$, $n_2$ and $n_3$ are each independently 0, or 1, or 2, or 3, or 4;

In some embodiments, $n_4$, $n_5$ and $n_6$ are all 0.

In some embodiments, $A^-$ is selected from the group consisting of halide ions, perchlorate, nitrate, sulfate, sulfhydrate, sulfite, phosphate, hydrophosphate, C1-C8 alkyl acid radicals, C1-C8 alkyl sulfonate, C1-C8 alkyl sulfate, and C1-C8 aryl sulfonate;

In some more specific embodiments, $A^-$ is preferably selected from sulfate, phosphate, acetate, propionate, chloride and bromide ions.

In some embodiments, when $R_1$ is hydrogen and $R_2$ is

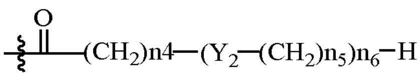

substituted by one or more

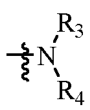

or

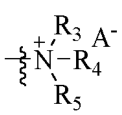

,

the chiral aryl propionic acid derivative of the present invention can form an inner salt or an intermolecular salt under proper conditions;

In some embodiments, when $R_1$ is hydrogen and $R_2$ is

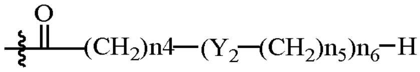

substituted by one or more

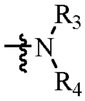 or 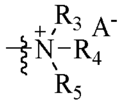 ,

the chiral aryl propionic acid derivative of the present invention can form salts with other anions under proper conditions, and the anions are as described above.

In some embodiments, when $R_2$ is hydrogen and $R_1$ is

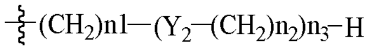

substituted by one or more

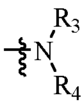 or 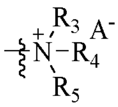 ,

the chiral aryl propionic acid derivative of the present invention can form salts with other anions under proper conditions, and the anions are as described above;

In some embodiments, when $R_1$ is

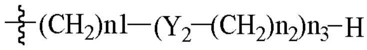

substituted by one or more

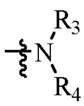 or 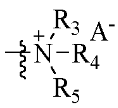

and $R_2$ is

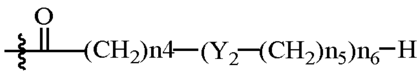

substituted by one or more

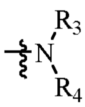 or 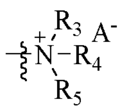 ,

the chiral aryl propionic acid derivative of the present invention can form salts with other anions under proper conditions, and the anions are as described above.

In some embodiments, the chiral aryl propionic acid derivative provided in the present invention is selected from the following compounds:

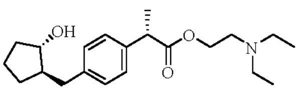 , 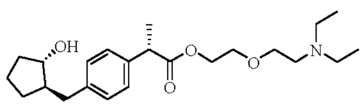 , 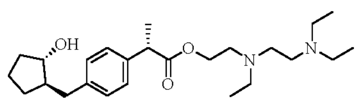 ,

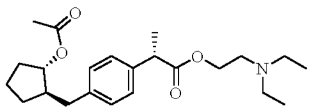, 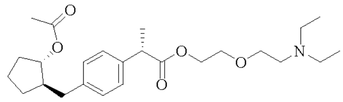, 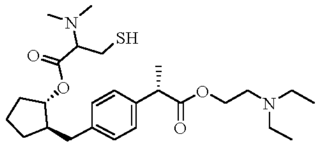,

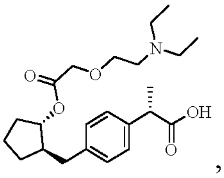, 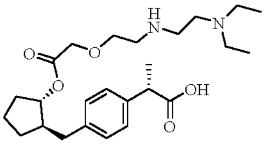, 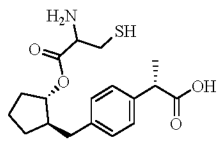,

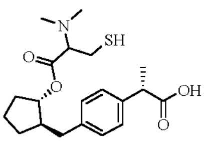, 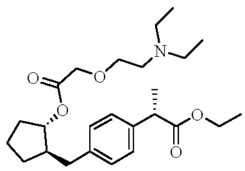, 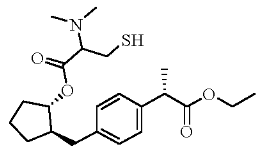,

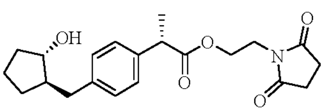, 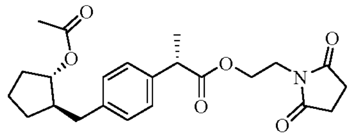, 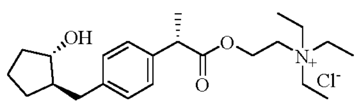,

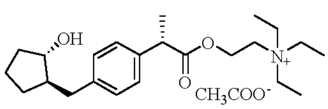, 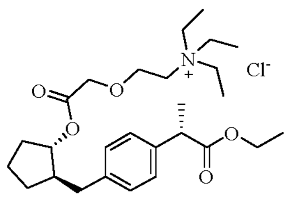, 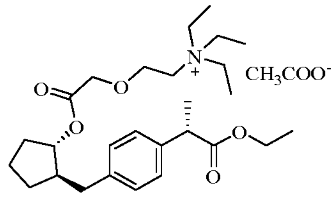,

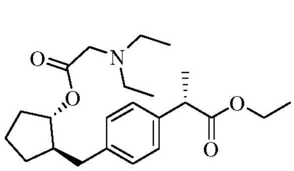, 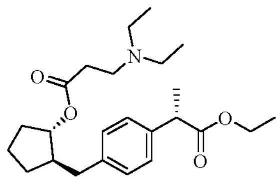, 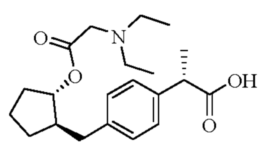,

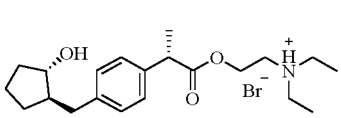, 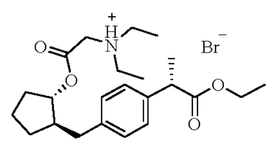, 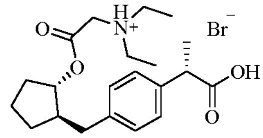,

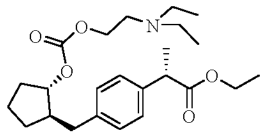, 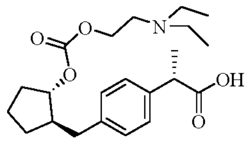,

or a pharmaceutically acceptable salt thereof.

In another aspect, the present invention provides a pharmaceutical composition comprising the above mentioned chiral aryl propionic acid derivative, a tautomer, a solvate or a pharmaceutically acceptable salt thereof.

The present invention discloses a pharmaceutical composition which is composed of the chiral aryl propionic acid

derivative, a tautomer, a solvate or a pharmaceutically acceptable salt thereof as described in the present invention as an active ingredient or a main active ingredient in combination with a pharmaceutically acceptable carrier.

The chiral aryl propionic acid derivative of the present invention can be prepared into a pharmaceutical composition. The prepared pharmaceutical composition is administrated to a patient in multiple properly selected administration routes including gastrointestinal local administration, for example, the chiral aryl propionic acid derivative can be prepared into topical preparations for skins, ophthalmic preparations, inhalation preparations and the like.

In some examples of the present invention, the chiral aryl propionic acid derivative of the present invention and lactose are mixed and smashed, and therefore prepared into inhalants.

In some examples of the present invention, the chiral aryl propionic acid derivative of the present invention and a proper amount of surfactants and osmotic pressure regulators are jointly dissolved and then prepared into a solution for inhalation.

In some examples of the present invention, the chiral aryl propionic acid derivative of the present invention a proper amount of surfactants and the like are jointly prepared into topical preparations for skins.

In some examples of the present invention, the chiral aryl propionic acid derivative of the present invention and proper accessories and the like are jointly prepared into ophthalmic preparations.

In a third aspect, the compound of the present invention has high bioavailability when it is administrated gastroenterally/parenterally and can be rapidly transformed into an active ingredient, and especially when it is administrated endermically or orally, it has a better effect.

The compound of the present invention has a better anti-rheumatoid arthritis effect. The compound of the present invention has a significant inhibitory effect on the proliferation of INF-$\alpha$ induced human rheumatoid arthritis fibroblast-like synovial cells (HFLS-RA), and can significantly inhibit the expression of inflammatory cytokines, exhibiting higher activity, and it is expected to reduce the administration dose and lower adverse reactions.

In addition, it is unexpectedly found that the compound of the present invention is highly distributed at joints after being orally administrated. The further study shows that the compound of the present invention has better joint fluid distribution and better therapeutic effects when externally applied on the skin, and can be used as a locally administrated non-steroidal anti-inflammatory drug.

In a fourth aspect, the present invention provides use of the above mentioned chiral aryl propionic acid derivative, a tautomer, a solvate or a pharmaceutically acceptable salt thereof as a non-steroidal anti-inflammatory drug, which is mainly useful for anti-inflammatory and analgesic treatment of conditions such as arthritis, rheumatoid arthritis, lumbago, scapulohumeral periarthritis and neck-shoulder-wrist syndrome, etc., as well as postoperative anti-inflammatory and analgesic treatment and antipyretic and analgesic treatment for acute respiratory inflammation.

## BRIEF DESCRIPTION OF THE DRAWINGS

FIG. 1 shows the comparison of distribution of control 1, control 2 and compounds DSC4813 and DSC4821 of the present invention at joints.

## DETAILED DESCRIPTION

The present invention can be more comprehensively understood by those skilled in the art through the following examples, which will not limit the present invention in any ways. The structures of all the compounds are determined by MS or $^1$H NMR.

### **Example 1: Synthesis** of trans-OH **metabolite**

A compound trans-OH metabolite was synthesized with reference to the literature (Mandai, T. and T. Yamakawa (2000). "An Efficient Synthesis of (2S)-2-[4-((1R,2S)-2-Hydroxycyclopentylmethyl)phenyl] propionic Acid." Synlett 2000(06): 0862-0864.), mp 87-88°C, $[M-H]^-$=247.16.$^1$H NMR (300 MHz, $CDCl_3$) $\delta$:1.20-1.32(m, 1H), 1.41-1.82(m, 7H), 1.88-2.06 (m, 2H), 2.46 (dd,1H), 2.75 (dd, 1H), 3.69 (q, 1H), 3.86-3.95 (m, 1H), 7.10-7.24 (m, 4H).

### Example 2: Synthesis of DSC4801

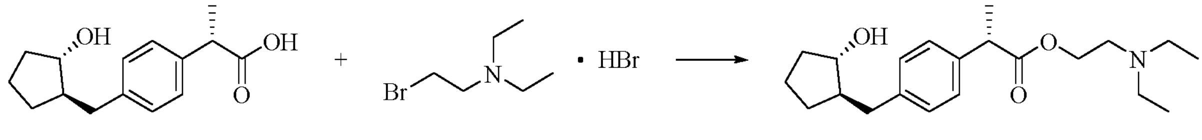

trans-OH metabolite 1 DSC4801

0.8 g of compound trans-OH metabolite was added into 10 mL of N,N-dimethylformamide (DMF), the system was cooled to 0°C, 1.01 g of compound 1 (2-bromo-N,N-diethylethylamine hydrobromide) and 0.85 g of sodium carbonate were added thereto, the system was stirred for 18 h. Water was added to precipitate out a solid, the solid was filtrated and then added into 20 mL of dichloromethane, water was added for liquid separation, the organic phase was concentrated to dryness, and then purified via a silica gel column to obtain 0.72 g of compound DSC4801, with a yield of 64%, $[M+H]^+$=348.33, $^1H$ NMR (300 MHz, $CDCl_3$) δ:0.95(t,6H),1.19-1.30(m, 1H), 1.41-1.71 (m, 6H),1.75-1.85 (m, 1H), 1.94-2.14 (m, 2H), 2.44 (dd,1H),2.51(m,4H), 2.78 (m, 3H), 3.74 (q, 1H), 3.86-4.05 (m, 1H),4.26(t,2H), 7.11-7.21 (m, 4H).

**Example 3: Synthesis of DSC4804**

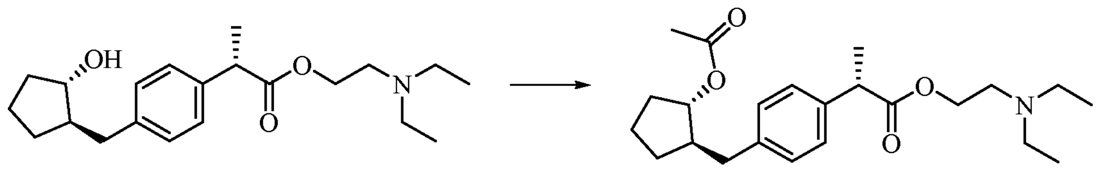

DSC4801 DSC4804

0.45 g of compound DSC4801, 0.15 g of pyridine and 10 mL of acetonitrile were added into a reaction flask, the reaction mixture was heated to 35°C, then 0.2 g of acetic anhydride was added to the above reaction mixture at 35-40°C, and then the mixture was reacted for 4 h at 50°C, the system was concentrated to dryness and then cooled to room temperature, 15 mL of dichloromethane and 15 mL of water were added into the system for liquid separation, and the organic phase was concentrated to dryness, and then purified via a silica gel column to obtain 0.39 g of compound DSC4804, with a yield of 77%; $[M+H]^+$=390.19, $^1H$ NMR (300 MHz, $CDCl_3$) δ:1.08 (t,6H),1.17-1.30(m, 1H), 1.45-1.86 (m, 7H), 1.94-2.11 (m, 5H), 2.49 (dd,1H),2.53(m,4H), 2.75 (m, 3H), 3.76 (q, 1H), 3.81-3.98 (m, 1H),4.3(t,2H), 7.07-7.20 (m, 4H).

**Example 4: Synthesis of DSC4807**

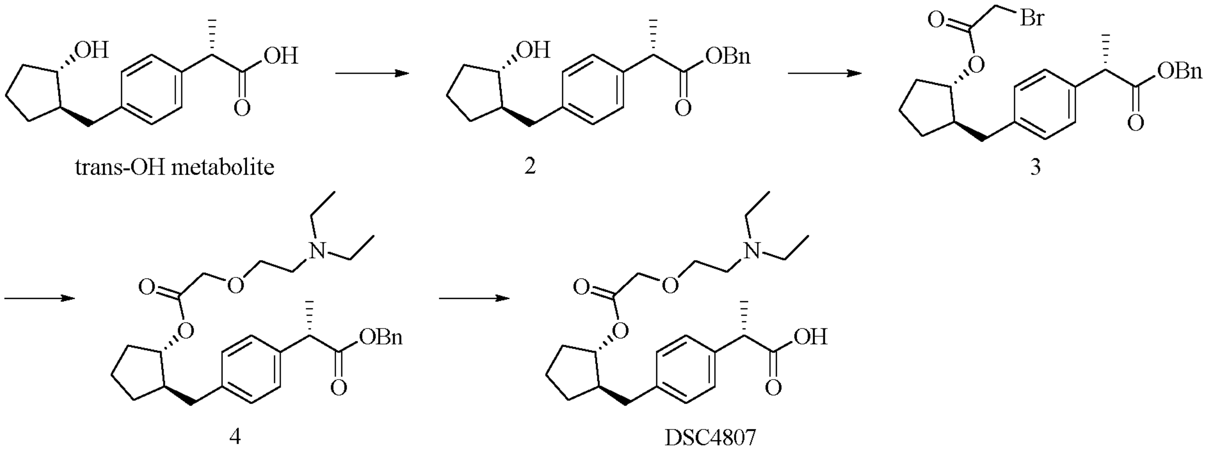

trans-OH metabolite 2 3

4 DSC4807

Synthesis of compound 2

1.0 g of compound trans-OH metabolite was added into 10 mL of N,N-dimethylformamide (DMF), the system was

cooled to 0°C, 0.83 g of benzyl bromide and 0.55 g of sodium carbonate were added thereto, the system was stirred for 3 h. Water was added to the system to precipitate out a solid, the solid was filtered and then added into 20 mL of dimethylformamide, then water was added for liquid separation, and the organic phase was concentrated to dryness, and then purified via a silica gel column to obtain 1.12 g of compound 2, with a yield of 82%; $[M+H]^+$=339.10;

Synthesis of compound 3

1.0 g of compound 2, 0.3 g of pyridine and 20 mL of anhydrous dichloromethane were added into a reaction flask, the reaction mixture was cooled to 0°C, then 0.66 g of bromoacetyl bromide was added, the reaction system was reacted for 12 h at room temperature, water was added into the above system for liquid separation, the organic phase was concentrated to dryness, and thenpurified via a silica gel column to obtain 1.06 g of compound 3, with a yield of 78%; $[M+H]^+$=459.21;

Synthesis of compound 4

0.2 g of compound diethylaminoethanol and 15 mL of anhydrous tetrahydrofuran were added into a reaction flask, the system was cooled to 0°C, and 0.05 g of sodium hydride (60%) was added, and then the system was stirred for 30 minutes. 0.71 g of compound 3 was added, the reaction mixture was heated to 35°C to react for 4 h, the above system was concentrated to dryness and cooled to room temperature, 15 mL of dichloromethane and 15 mL of water were added for liquid separation, the organic phase was concentrated to dryness, and then purified via a silica gel column to obtain 0.63 g of compound 4, with a yield of 75%; $[M+H]^+$=496.34;

Synthesis of compound DSC4807

1.19 g of 10% palladium carbon was added into 0.5 g of compound 4, 10 mL of methanol was added into the above system, then the system was connected with a hydrogen balloon, and heated to 30°C to react for 8 h. The system was filtered, the filtrate was concentrated to dryness, and then purified via a silica gel column to obtain 0.36 g of compound DSC4807, with a yield of 88%; $[M-H]^-$=404.29.

**Example 5: Synthesis of DSC4806**

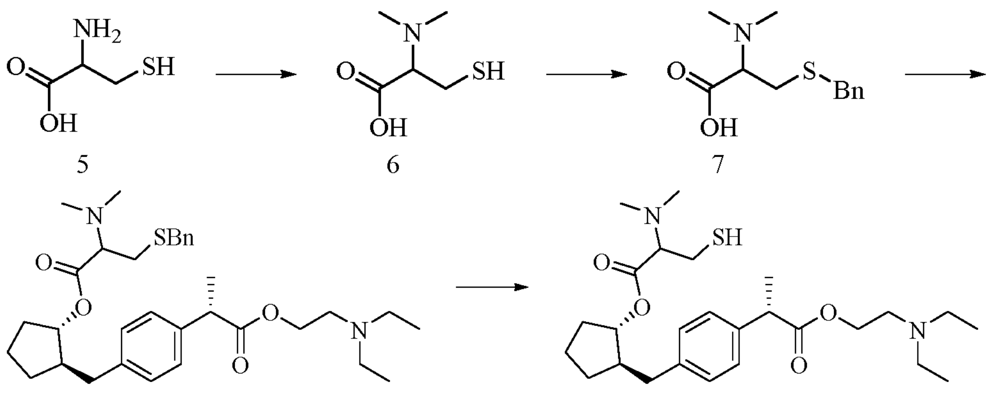

5 6 7

8 DSC4806

Synthesis of compound 6

Compound 6 was synthesized with reference to the method in the literature ("Characterization of N,N-dimethyl amino acids by electrospray ionization-tandem mass spectrometry." J. Mass Spectrom. 2015, 50, 771-781.

Synthesis of compound 7

1.2 g of compound 6 and 30 mL of ethanol were added into a reaction flask, then 10 mL of 2N sodium hydroxide solution was added into the above system, and then 1.51 g of benzyl bromide was added with intense stirring. After the system was reacted for 2 h at room temperature, concentrated hydrochloric acid was added to adjust pH to be neutral, a

solid was precipitated out and filtered, the filter cake was washed with water and ethanol successively, and the system was dried to obtain 1.48 g of compound 7, with a yield of 77%, $[M-H]^-$=238.25.

Synthesis of compound 8

1.2 g of compound 7 and 15 mL of dichloromethane were added into a reaction flask, 1 g of thionyl chloride was added into the above system, then the mixture was reacted for 3 h at room temperature, the system was concentrated to dryness, and then 30 mL of dichloromethane was added again, and then the system was concentrated to dryness. 15 mL of dichloromethane and 1 mL of triethylamine were added into the system, 1.74 g of compound DSC4801 was added thereto, the system was heated to 40°C to react for 5 h, and then it was concentrated to dryness, and then purified via a silica gel column to obtain 1.83 g of compound 8, with a yield of 64%, $[M+H]^+$=569.33;

Synthesis of compound DSC4806

0.25 g of 5% palladium/carbon was added into 0.6 g of compound 8, 10 mL of methanol was added into the above system, then the system was connected with a hydrogen balloon, and then the system was heated to 30°C to react for 8 h. The system was filtered, and the filtrate was concentrated to dryness, and then purified via a silica gel column to obtain 0.43 g of compound DSC4806, with a yield of 85%; $[M+H]^+$=479.29.

**Example 6: Synthesis of DSC4815 and DSC4816**

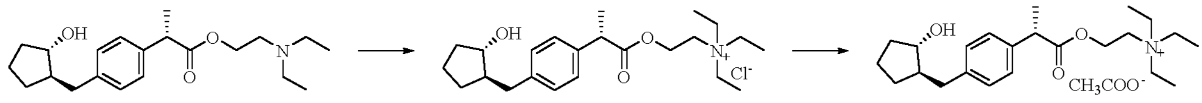

DSC4801 DSC4815 DSC4816

Synthesis of compound DSC4815

20 mL of tetrahydrofuran and 2.6 g of compound DSC4801 were added into a reaction flask successively at room temperature under the protection of nitrogen, and 8 mL of 0.54 g of chloroethane in tetrahydrofuran was slowly added thereto. After the addition was completed, the above system was heated to reflux and then reacted for 30 minutes. After the reaction was completed, the system was cooled to 0-10°C, filtered and dried to obtain a crude product. The crude product was re-crystallized using a methanol/acetone mixed solvent to obtain 1.33 g of compound DSC4815, with a yield of 43%, $[M+H]^+$=376.03;

Synthesis of compound DSC4816

1 g of compound DSC4815 and 5 mL of anhydrous ethanol were added into a reaction flask and heated at 50°C, then 0.45 g of silver acetate was added thereto, the reaction mixture was stirred and reacted for 3 h and then subjected to hot filtration, the filtrate was cooled to about 10°C, and then 13 mL of methyl tert-butyl ether was added for crystallization, 0.39 g of compound DSC4816 was obtained, with a yield of 37%, $[M+H]^+$=376.03, $[M-H]^-$=59.01.

**Example 7: Synthesis of DSC4821**

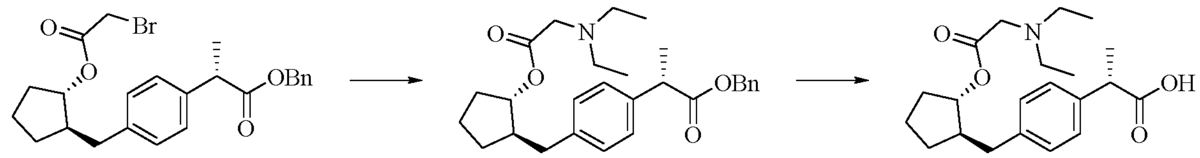

3 11 DSC4821

Synthesis of compound 11

0.18 g of compound diethylamine hydrochloride, 0.5 g of compound 3 and 15 mL of anhydrous tetrahydrofuran were added into a reaction flask, the system was cooled to 0°C, and 0.7 mL of triethylamine was added, and then the reaction mixture was heated to 35°C to react for 4 h. The system was concentrated to dryness and cooled to room temperature, and 15 mL of dichloromethane and 15 mL of water were added into the above system for liquid separation, the organic phase was concentrated to dryness, and then purified via a silica gel column to obtain 0.34 g of compound 11, with a yield of 69%; $[M+H]^+$=452.34;

Synthesis of compound DSC4821

50 mg of palladium/carbon (10%) was added into 0.3 g of compound 11, 10 mL of methanol was added into the above system, and then the system was connected with a hydrogen balloon. The system was heated to 30°C to react for 8 h. The system was filtered, and the filtrate was concentrated to dryness, and then purified via a silica gel column to obtain 0.2 g of compound DSC4821, with a yield of 84%; $[M-H]^-$=360.29, $^1H$ NMR (300 MHz, $CDCl_3$) δ:0.99(t,6H),1.22-1.30(m, 1H), 1.37-1.66 (m, 3H), 1.45 (d,3H), 1.70-1.79 (m, 1H), 1.83-2.02 (m, 2H), 2.43-2.66 (m, 6H), 2.75 (dd,1H),3.15(s, 2H), 3.65 (q, 1H), 3.76-3.88 (m, 1H), 7.08-7.27 (m, 4H).

**Example 8: Synthesis of DSC4826**

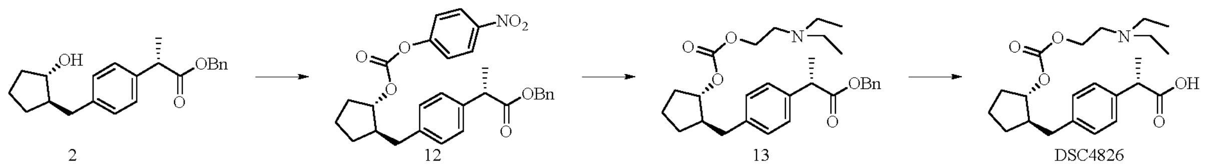

Synthesis of compound 12

0.7 g of compound 2, 1.25 g of p-nitrophenyl chloroformate, 1 mL of triethylamine and 10 mL of tetrahydrofuran were added into a reaction flask. The above system was reacted for 1 h at room temperature. The system was concentrated to dryness, then ethyl acetate and water were added for extraction, the organic phase was concentrated to dryness, and then purified via a silica gel column to obtain 0.8 g of compound 12, with a yield 77%, $[M+H]^+$=504.21;

Synthesis of compound 13

0.13 g of compound diethylaminoethanol and 15 mL of anhydrous tetrahydrofuran were added into a reaction flask, the above system was cooled to 0°C, 65 mg of sodium hydride (60%) was added, and then the system was stirred for 30 min. 0.55 g of compound 12 was added, the reaction mixture was heated to 35°C to react for 4 h, the above system was concentrated to dryness and then cooled to room temperature, then 15 mL of dichloromethane and 15 mL of water were added for liquid separation, the organic phase was concentrated to dryness, and then purified via a silica gel column to obtain 0.37 g of compound 13, with a yield of 71%; $[M+H]^+$=482.39;

Synthesis of compound DSC4826

0.18 g of compound DSC4826 was synthesized by using compound 13 as a raw material with reference to the synthesis method of compound DSC4821, with a yield of 84%, $[M-H]^-$=390.33, $^1H$ NMR (300 MHz, $CDCl_3$) δ:1.01(t,6H), 1.22-1.30(m, 1H), 1.38-1.82 (m, 7H), 1.91-2.06 (m, 2H), 2.46 (dd,1H), 2.51-2.55(m,4H), 2.75-2.88(m, 3H), 3.71 (q,1H), 3.86-3.91(m, 1H), 4.23-4.25(t,2H), 7.05-7.20 (m, 4H).

The following example compounds were synthesized according to the same method as those in the above examples by using commercially available compounds or intermediate compounds appropriately synthesized from commercially available compounds.

| | | |
|---|---|---|
| 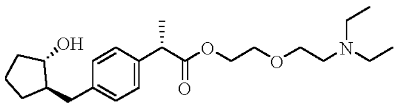 DSC4802 [M+H]$^+$=392.02 | 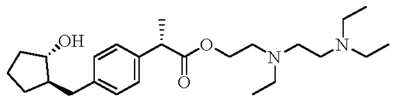 DSC4803 [M+H]$^+$=419.51 | 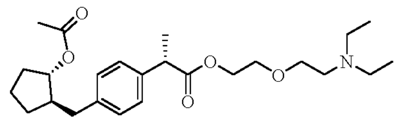 DSC4805 [M+H]$^+$=434.38 |
| 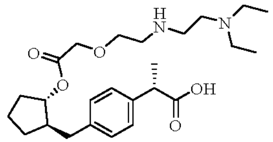 DSC4808 [M-H]$^-$=447.17 | 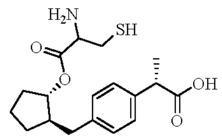 DSC4809 [M-H]$^-$=350.48 | 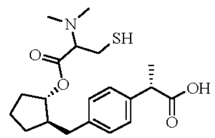 DSC4810 [M-H]$^-$=378.33 |
| 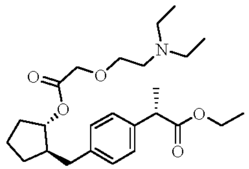 DSC4811 [M+H]$^+$=434.32 | 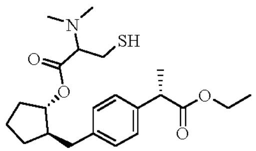 DSC4812 [M+H]$^+$=408.22 | 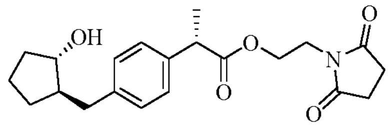 DSC4813 [M+H]$^+$=374.33 |
| 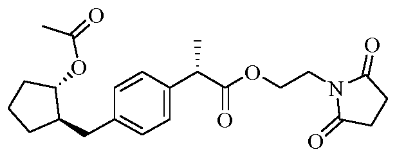 DSC4814 [M+H]$^+$=416.54 | 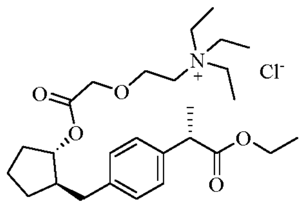 DSC4817 [M+H]$^+$=462.12 | 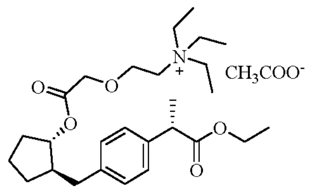 DSC4818 [M+H]$^+$=462.12, [M-H]$^-$=59.01 |

(continued)

| | | |
|---|---|---|
| 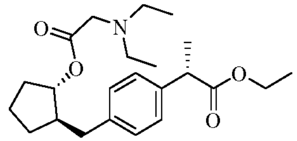 DSC4819 $[M+H]^+$=390.36 | 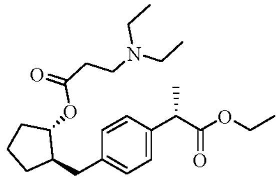 DSC4820 $[M+H]^+$=404.38 | 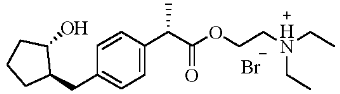 DSC4822 $[M+H]^+$=348.35 |
| 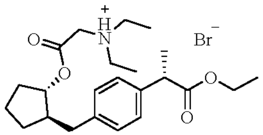 DSC4823 $[M+H]^+$=390.36 | 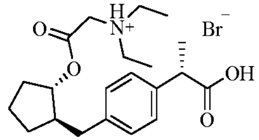 DSC4824 $[M+H]^+$=362.33 | 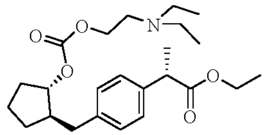 DSC4825 $[M+H]^+$=420.39 |

**Synthesis of comparative examples**

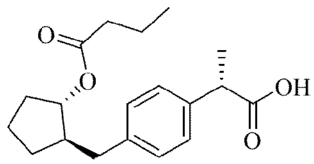

Control 1

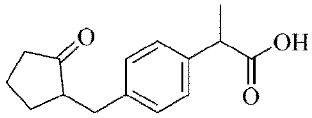

Control 2

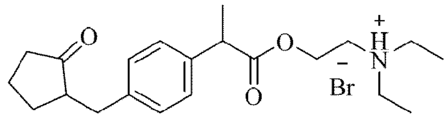

Control 3

The synthesis of control 1 was achieved with reference to the synthesis methods of example compounds. $[M+H]^-$ =317.37, $^1$H NMR (300 MHz, $CDCl_3$) δ:0.78(t, 3H),1.06-1.29 (m, 3H), 1.38-1.76(m,7H), 1.81-2.02 (m, 2H),2.11(t,2H), 2.47 (dd,1H), 2.79 (dd, 1H), 3.63 (q, 1H), 3.85-3.95 (m, 1H), 7.13-7.27 (m, 4H).
Control 2 was commercially available.
The synthesis of control 3 was synthesized with reference to the synthesis methods in examples of this patent and patent CN103705496B.

**Example 9: Effect on HFLS-RA cell proliferation activity**

Grouping and sample concentration: the experiment was divided into blank control groups (2 groups in total, including group 1 and group 2, where group 1 was used for CCK-8 detection at 0 h, and group 2 was used for CCK-8 detection at 72 h; both of the 2 groups did not contain test samples and TNF-α), TNF-α groups (only TNF-α was added without test samples, and the final concentration of TNF-α was 10 ng/mL), test sample groups (test samples were DSC4801-DSC4826 and controls 1 to 3, the structures of controls 1 to 3 can make reference to synthesis parts in comparative examples, and the final concentrations of various test samples for cell incubation and of TNT-α were 100 μg/mL and 10 ng/mL, respectively) and positive control groups (the final concentrations of methotrexate for cell incubation and of TNT-α were 1 μg/mL and 10 ng/mL, respectively).
Co-incubation: HFLS-RA cells in a logarithmic growth phase were inoculated in a 96-well culture plate at a density of 3 x $10^4$ cells/ml with an inoculation volume of each well being 100 μL, and cultured for 24 h in a 37°C and 5%$CO_2$ incubator (the cell adhesion convergence degree reached about 25%) after inoculation, and then a proper amount of sample solutions was added according to the grouping and final concentrations of samples, with 3 duplicated wells set in each group. Before sampling (0 h), group 1 in blank control group was subjected to CCK-8 detection, and the rest groups were cultured for 72 h in an incubator and then subjected to CCK-8 detection.
Detection and calculation: the culture solution was discarded, and 100 μL of culture medium containing a 10% CCK-8 solution was added into each well and incubated for 2 h at 37°C. The light absorption value (OD value) was measured at 450 nm using a microplate reader, and the cell proliferation rate was calculated according to the following formula. The results are as shown in Table 1.

$$\text{Calculation formula: cell proliferation rate}=[OD_{(72h)}- OD_{(0h)}]/ OD_{(0h)} \times 100\%,$$

where, $OD(_{0h})$ represents the OD value at 0h, and $OD(_{0h})$ represents the OD value at 72 h.

Table 1: HFLS-RA cell proliferation rate $(\bar{x}\pm s)$ 72 h after treatment

| Group | Cell proliferation rate | Group | Cell proliferation rate | Group | Cell proliferation rate |
|---|---|---|---|---|---|
| Blank control group | 61.32±2.01 | TNF-α group | 88.57±3.66 | Negative control group | 18.37±1.32 |
| DSC4801 | 31.18±1.82 | DSC4802 | 27.26±2.41 | DSC4803 | 30.17±1.85 |
| DSC4804 | 29.36±2.33 | DSC4805 | 29.28±1.92 | DSC4806 | 24.19±2.24 |
| DSC4807 | 22.23±3.04 | DSC4808 | 23.11±2.25 | DSC4809 | 22.47±1.99 |
| DSC4810 | 24.95±2.65 | DSC4811 | 22.37±1.78 | DSC4812 | 23.81±2.21 |
| DSC4813 | 22.34±2.72 | DSC4814 | 24.36±1.99 | DSC4815 | 28.31±2.26 |

(continued)

| Group | Cell proliferation rate | Group | Cell proliferation rate | Group | Cell proliferation rate |
|---|---|---|---|---|---|
| DSC4816 | 29.71±2.83 | DSC4817 | 23.32±2.46 | DSC4818 | 22.54±2.14 |
| DSC4819 | 20.36±2.10 | DSC4820 | 21.11±3.56 | DSC4821 | 20.96±1.85 |
| DSC4822 | 31.54±2.23 | DSC4823 | 22.78±1.87 | DSC4824 | 22.17±2.29 |
| DSC4825 | 22.53±2.66 | DSC4826 | 21.43±1.94 | Control 1 | 39.33±3.46 |
| Control 2 | 48.25±3.21 | Control 3 | 44.31±4.15 | | |

It can be seen from Table 1 that compared with blank control group, TNF-α group significantly promotes HFLS-RA cell proliferation (P<0.001); compared with TNF-α group, both of positive control group and test sample group (compounds DSC4801-DSC4826 of the present invention, and controls 1 to 3) significantly inhibit TNF-α induced HFLS-RA cell proliferation (P<0.001); compared with control 1 group, control 2 group and control 3 group, the compound of the present invention has significantly higher inhibition level to the TNF-α induced HFLS-RA cell proliferation (P<0.001). The results show that the compound of the present invention can exert the anti-inflammatory effect through inhibition of HFLS-RA cell proliferation, and is obviously superior to control compounds 1, 2 and 3.

**Example 10: Effect on HFLS-RA cytokine secretion**

The experiment was divided into a blank control group (containing no test samples or TNF-α), a TNF-α group (the final concentration of TNF-α for cell incubation was 10 ng/mL), test sample groups (8 groups in total, including DSC4801 group, DSC4807 group, DSC4810 group, DSC4813 group, DSC4821 group and DSC4826 group as well as control 1 group, control 3 group, the final concentrations of test samples for cell incubation and of TNT-α were 100 μg/mL and 10 ng/mL, respectively).

HFLS-RA cells in a logarithmic growth phase were inoculated in a 6-well culture plate at a density of 5 x$10^4$cell/ml with an inoculation volume of each well being 200 μL, and cultured for 2 h in a 37°C and 5%$CO_2$ incubator after inoculation, and then a proper amount of sample solutions was added according to the grouping and the final concentrations of samples, with 3 duplicated wells set in each group. Cell supernatant was collected after further culture of 48 h, and the contents of IL-6 and IL-8 in the supernatant were detected using an ELISA kit. The results are as shown in Table 2.

Table 2: Effect ($\bar{x}$±s) of samples on HFLS-RA cytokines IL-6 and IL-8 expression

| Group | IL-6 | IL-8 | Group | IL-6 | IL-8 |
|---|---|---|---|---|---|
| Blank control group | 44.51±0.58 | 107.35±3.01 | TNF-α group | 93.85±1.74 | 162.08±7.35 |
| DSC4801 | 46.26±0.35 | 119.08±4.55 | DSC4807 | 48.01±0.77 | 121.66±4.78 |
| DSC4810 | 47.88±0.72 | 118.57±3.92 | DSC4813 | 46.82±0.63 | 119.48±4.07 |
| DSC4821 | 46.58±0.61 | 117.72±3.43 | DSC4826 | 47.59±0.45 | 117.77±4.23 |
| Control 1 | 67.45±2.68 | 131.21±6.88 | Control 3 | 74.02±3.53 | 135.76±7.61 |

It can be seen from Table 2 that compared with blank control group, TNF-α can significantly induce the expression of HFLS-RA cell IL-6 and IL-8 inflammatory factors (P<0.001); compared with TNF-α group, control 1 group and control 3 group, the compound of the present invention can significantly inhibit the expression of TNF-α induced HFLS-RA cell IL-6 and IL-8 inflammatory factors (P<0.001).The results show that the compound of the present invention can exert anti-inflammatory effect by inhibiting the expression of IL-6 and IL-8 inflammatory factors, and is obviously superior to control compounds 1 and 3.

**Example 11: Joint tissue distribution**

36 healthy male SD rats with a body weight of 200 ± 20 g per rat were fed under conditions of a room temperature of 20-26°C, a humidity of 40%-70% and a light and dark cycle of 12 h/12 h, the rats were fed at liberty in the process of feeding. After adaptive feeding for 3 days, the SD rats were randomly divided into 4 groups (A/B/C/D groups), with 9 rats per group, each group was further randomly divided into 3 time groups, with 3 rats per time group. A suspension (the test

samples were suspended with 1% MS, respectively) of the test sample was given to each group via oral gavage, wherein group A was administrated with 3 mg/kg of DSC4813, group B was administrated with 2.94 mg/kg of DSC4821, group C was administrated with 2.47 mg/kg of control 1, and group D was administrated with 2 mg/kg of control 2 (loxoprofen). 3 rats were anesthetized and euthanized in each group at 0.5 h, 1 h and 2 h, respectively after administration. The left and right ankle joints of hind legs were dissected, and the skin at the joints was peeled off. The surrounding tissues of the ankle joints (including inner and outer collateral ligaments, inner and outer triangular ligaments, fascia, tendons, joint synovium, etc.) were weighed. The joint tissue samples were ultrasonically extracted with 10 mL of methanol for 20 min after being cut into pieces, after the extraction was completed, the joint tissue samples were put in EP tubes, sealed and stored at -20°C for later use. To-be-detected substances were detected by LC-MS/MS (compound trans-OH metabolite was detected for groups A/B/C, loxoprofen and all isomers whose carbonyl was reduced to OH for group D, and the results of group D were a sum of concentrations of loxoprofen and all isomers whose carbonyl was reduced to OH). The results are as shown in FIG. 1.

It can be seen from FIG. 1 that compared with control 1 and control 2, the distribution of the compounds DSC4813 and DSC4821 of the present invention at joints is significantly improved after oral gavage administration ($P<0.001$). Therefore, it is inferred that the compounds DSC4813 and DSC4821 of the present invention have strong an anti-arthritis effect, and are superior to control compounds 1 and 2.

Although the present invention has been disclosed in preferred embodiments as described above, it is not intended to limit the present invention. Any person skilled in the art may make slight modifications and improvements without departing from the spirit and scope of the present invention. Therefore, the scope of protection of the present invention shall be determined by the appended claims.

## Claims

1. A chiral aryl propionic acid derivative as shown in formula (I), and a tautomer, a solvate or a pharmaceutically acceptable salt thereof:

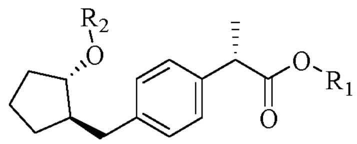

(I)

in the formula (I), $R_1$ is H, or is selected from

$$-(CH_2)n1-(Y_2-(CH_2)n_2)n_3-H$$

substituted by one or more

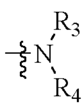 or 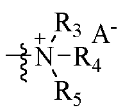

or unsubstituted

$$-(CH_2)n1-(Y_2-(CH_2)n_2)n_3-H;$$

$R_2$ is H, or is selected from

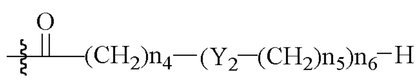

substituted by one or more

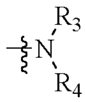 or 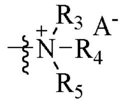

or unsubstituted

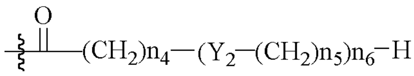;

and when $R_1$ is H, $R_2$ is not H;
$Y_2$ is O, or $N(R_6)$, or S;
$n_1$, $n_2$, $n_3$, $n_4$, $n_5$ and $n_6$ are each independently 0, 1, 2, 3, 4, 5, 6, 7, 8, 9, and 10;
$R_3$, $R_4$ and $R_5$ are each independently H, C1-C20 hydrocarbyl, or C1-C20 alkyl carbonyl, or $R_3$ and $R_4$ form a ring together with a nitrogen atom to which they are attached;
$R_6$ is H, or C1-C20 hydrocarbyl; and
$A^-$ represents an acceptable inorganic or organic anion.

**2.** The chiral aryl propionic acid derivative, and a tautomer, a solvate or a pharmaceutically acceptable salt thereof according to claim 1, wherein $R_1$ is H, and $R_2$ is

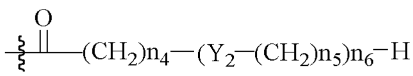

substituted by one or more

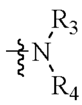 or 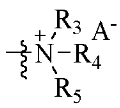

or unsubstituted

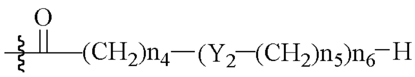,

wherein $n_4$, $n_5$ and $n_6$ are each independently 0, 1, 2, 3, 4, 5, 6, 7, 8, 9, and 10.

**3.** The chiral aryl propionic acid derivative, and a tautomer, a solvate or a pharmaceutically acceptable salt thereof according to claims 1 and 2, wherein $R_1$ is H, and $R_2$ is

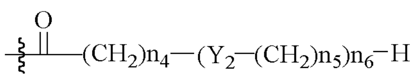

substituted by one or more

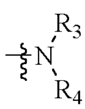 or 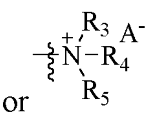

or unsubstituted

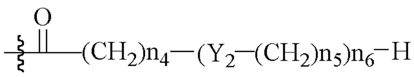,

wherein $n_4$, $n_5$ and $n_6$ are each independently 0, 1, 2, 3, and 4.

**4.** The chiral aryl propionic acid derivative, and a tautomer, a solvate or a pharmaceutically acceptable salt thereof according to claim 1, wherein $R_2$ is H, and $R_1$ is

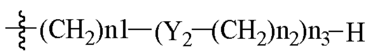

substituted by one or more

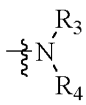 or 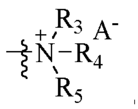

or unsubstituted

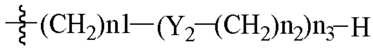,

wherein $n_1$, $n_2$ and $n_3$ are each independently 0, 1, 2, 3, 4, 5, 6, 7, 8, 9, and 10.

**5.** The chiral aryl propionic acid derivative, and a tautomer, a solvate or a pharmaceutically acceptable salt thereof according to claim 1 or 4, wherein $R_2$ is H, and $R_1$ is

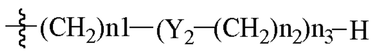

substituted by one or more

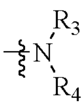 or 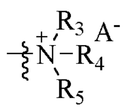

or unsubstituted

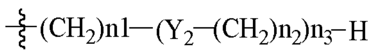,

wherein $n_1$, $n_2$ and $n_3$ are each independently 0, 1, 2, 3, and 4.

**6.** The chiral aryl propionic acid derivative, and a tautomer, a solvate or a pharmaceutically acceptable salt thereof according to any one of claims 1 to 5, wherein $R_3$, $R_4$ and $R_5$ are each independently H, or C1-C4 alkyl hydrocarbyl.

**7.** The chiral aryl propionic acid derivative according to claim 1, which is selected from the following compounds:

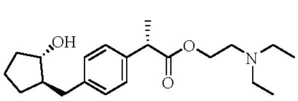, 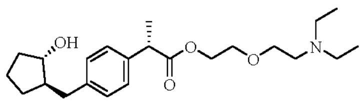, 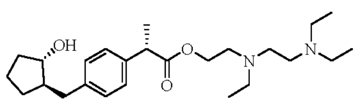,

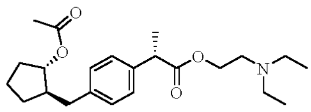 , 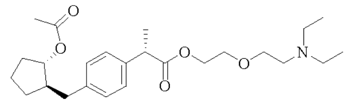 , 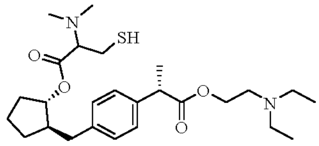 ,

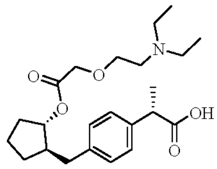 , 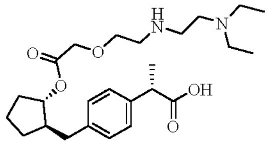 , 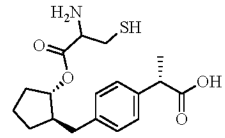 ,

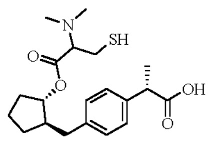 , 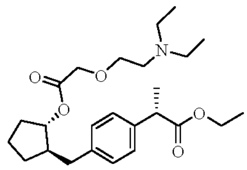 , 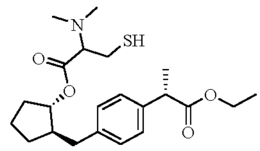 ,

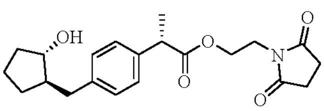 , 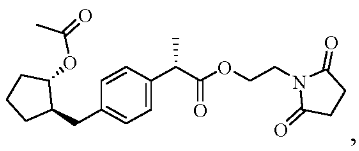 , 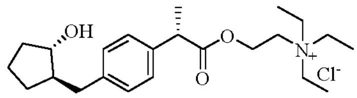 ,

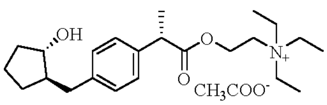 , 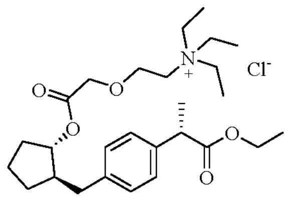 , 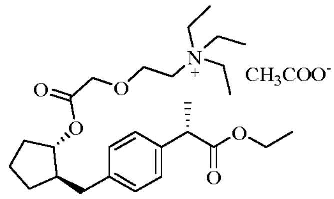 ,

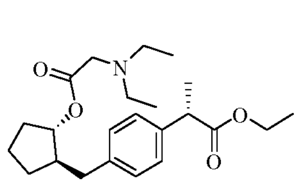 , 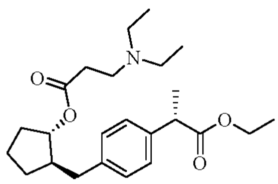 , 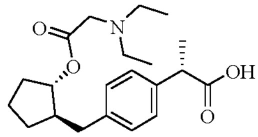 ,

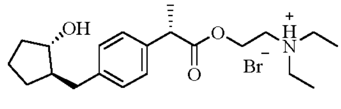 , 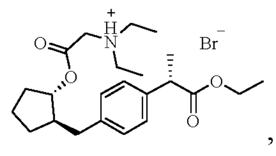 , 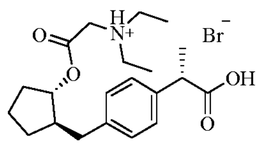 ,

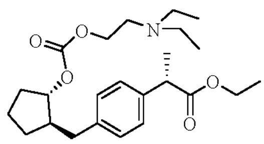 , 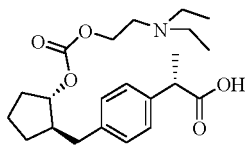 ,

or a pharmaceutically acceptable salt thereof.

**8.** The chiral aryl propionic acid derivative according to claims 1 to 7, wherein the pharmaceutically acceptable salt

comprises salts formed by the chiral aryl propionic acid derivative and anions, and the anions include but are not limited to fluoride ions, chloride ions, bromide ions, iodide ions, acetate ions, benzoate ions, citrate ions, tartrate ions, oxalate ions, malate ions, ascorbate ions, and fumarate ions, etc.

**9.** The chiral aryl propionic acid derivative according to claims 1 to 3, wherein the pharmaceutically acceptable salt comprises inner salts or intermolecular salts.

**10.** A pharmaceutical composition comprising the chiral aryl propionic acid derivative, and a tautomer, a solvate or a pharmaceutically acceptable salt thereof according to any one of claims 1 to 9.

**11.** Use of the chiral aryl propionic acid derivative, and a tautomer, a solvate or a pharmaceutically acceptable salt thereof according to any one of claims 1 to 9, or the pharmaceutical composition according to claim 10 in preparing antipyretic, analgesic and anti-inflammatory drugs.

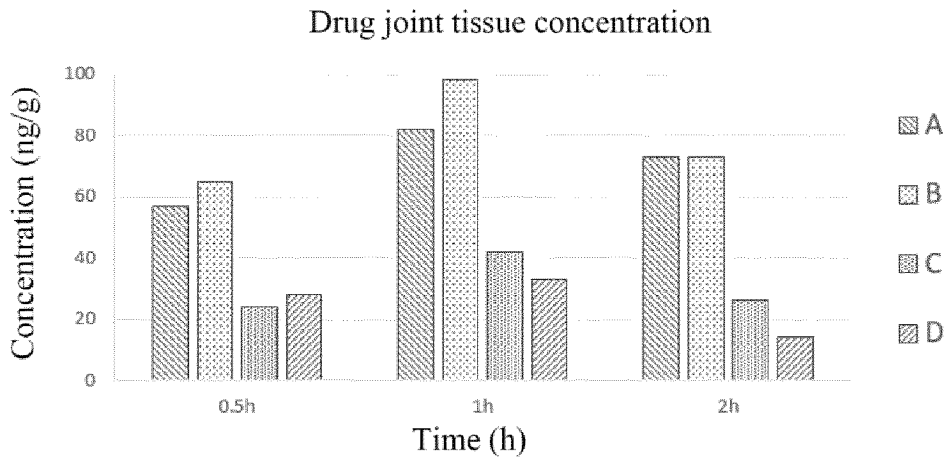
Drug joint tissue concentration
Concentration (ng/g)
100
80
60
40
20
0
0.5h
1h
2h
Time (h)
A
B
C
D

FIG. 1

**INTERNATIONAL SEARCH REPORT**

International application No.
**PCT/CN2023/088864**

**A. CLASSIFICATION OF SUBJECT MATTER**

C07C219/10(2006.01)i； C07C217/08(2006.01)i； C07C323/58(2006.01)i； C07C219/16(2006.01)i； C07C229/12(2006.01)i； C07D209/48(2006.01)i； A61K31/216(2006.01)i； A61K31/4035(2006.01)i； A61K31/223(2006.01)i； A61K31/265(2006.01)i； A61K31/22(2006.01)i； A61P29/00(2006.01)i

According to International Patent Classification (IPC) or to both national classification and IPC

**B. FIELDS SEARCHED**

Minimum documentation searched (classification system followed by classification symbols)

IPC:C07C C07D A61K A61P

Documentation searched other than minimum documentation to the extent that such documents are included in the fields searched

Electronic data base consulted during the international search (name of data base and, where practicable, search terms used)

CNTXT; CNABS; WPABS; DWPI; CJFD; CNKI; ENTXT; VEN; VCN; STN-REGISTRY; STN-CAPLUS; ISI-Web of Science; 超星读秀; DUXIU; 百度学术; BAIDU SCHOLAR: 迪斯凯威, 孟月垒, 洛索洛芬, 抗炎, 式I结构式检索, formula I structural formula search, loxoprofen, +inflammatory

**C. DOCUMENTS CONSIDERED TO BE RELEVANT**

| Category* | Citation of document, with indication, where appropriate, of the relevant passages | Relevant to claim No. |
|---|---|---|
| X | CN 106661061 A (NANJING HERON PHARMACEUTICAL SCIENCE AND TECHNOLOGY CO., LTD. et al.) 10 May 2017 (2017-05-10)<br>claims 1, 6, 9, and 10 | 1-3, 6-11 |
| X | WO 2014065577 A1 (HANMI FINE CHEMICAL CO., LTD.) 01 May 2014 (2014-05-01)<br>description, embodiment 3 | 1, 4, 5 |
| X | Shunji Naruto et al. "Structural determination of rat urinary metabolites of sodium 2-[4-(2-oxocyclopentylmethyl)phenyl]propionate dihydrate (loxoprofen sodium), a new antiinflammatory agent"<br>*Chemical and Pharmaceutical Bulletin,*<br>Vol. 32, No. 1, 25 January 1984 (1984-01-25), 258-267<br>ISSN: 0009-2363,<br>page 259, Chart 1, and page 260, paragraphs 3-4 | 1, 4, 5 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 August 2023** | **18 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/088864**

**C. DOCUMENTS CONSIDERED TO BE RELEVANT**

| Category* | Citation of document, with indication, where appropriate, of the relevant passages | Relevant to claim No. |
|---|---|---|
| X | Shunji Naruto et al. "Synthesis of the Eight Possible Optically Active Isomers of 2-[4-(2-Hydroxycyclopentylmethyl) phenyl] propionic Acid" *Chemical and Pharmaceutical Bulletin,* Vol. 31, No. 12, 25 December 1983 (1983-12-25), 4319-4323 ISSN: 0009-2363, page 4320, Chart 2, page 4321, last paragraph, and Table I | 1, 4, 5 |
| A | CN 111635309 A (SHAANXI SYNTHETIC PHARMACEUTICAL CO., LTD.) 08 September 2020 (2020-09-08) claims 1-5 | 1-11 |
| A | HAI, Ye et al. "HR1405-01, a Safe intravenous NSAID with superior anti-inflammatory and analgesic activities in preclinical trials" *European Journal of Medicinal Chemistry,* Vol. 235, No. 5, 18 March 2022 (2022-03-18), 114258 ISSN: 0223-5234, Scheme 1-2 | 1-11 |

Form PCT/ISA/210 (second sheet) (July 2022)

**INTERNATIONAL SEARCH REPORT**
**Information on patent family members**

International application No.
**PCT/CN2023/088864**

| Patent document cited in search report | | | Publication date (day/month/year) | Patent family member(s) | | | Publication date (day/month/year) |
|---|---|---|---|---|---|---|---|
| CN | 106661061 | A | 10 May 2017 | WO | 2017012556 | A1 | 26 January 2017 |
| | | | | CN | 106661061 | B | 11 June 2019 |
| WO | 2014065577 | A1 | 01 May 2014 | KR | 20140059872 | A | 16 May 2014 |
| | | | | KR | 101451171 | B1 | 16 October 2014 |
| CN | 111635309 | A | 08 September 2020 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103705496 B


### Non-patent literature cited in the description


- **MANDAI, T** ; **T. YAMAKAWA**. An Efficient Synthesis of (2S)-2-[4-((1R,2S)-2-Hydroxycyclopentylmethyl) phenyl] propionic Acid. *Synlett*, 2000 (06), 0862-0864
- Characterization of N,N-dimethyl amino acids by electrospray ionization-tandem mass spectrometry. *J. Mass Spectrom.*, 2015, vol. 50, 771-781